# EUROPEAN PATENT APPLICATION

(11) **EP 3 216 427 A1**
(43) Date of publication of application: **13.09.2017**
(21) Application number: 17150934.2
(22) Date of filing: 11.01.2017
(51) Int. Cl.: A61F 2/64, A61F 2/66, A61F 2/50

(54) **BUFFER MECHANISM OF ARTIFICIAL LIMB**

(30) Priority: 09.03.2016 TW 105203215 U
(71) Applicant: PRO LIMB International Corp., New Taipei City 238 (TW)
(72) Inventor: SHEN, Hsin-Fa, Shulin City, Taipei County 238 (TW)
(74) Representative: Viering, Jentschura & Partner mbB Patent- und Rechtsanwälte

(57) **Abstract**

A buffer mechanism of an artificial limb is provided, including a joint connecting base (20), an artificial limb connecting base (10), a first connecting rod (30) connected to a front side of the joint connecting base (20), a second connecting rod (40) connected to a rear side of the artificial limb connecting base (10), and a buffer cylinder (5, 60) disposed between the first connecting rod (30) and the second connecting rod (40) and having a sleeve (7, 61), a cylinder plug (6, 62) and a spring (8, 63). The cylinder plug (6, 62) is detachably disposed at one end of the sleeve (7, 61), the side surface of the first connecting rod (30) facing the second connecting rod (40) is disposed with a roller arm (50), an end of the roller arm (50) is pivotally connected with a roller (51), and the roller arm (50) contacts the end face of the cylinder plug (6, 62) by the roller (51). The present disclosure can reduce the wear rate of the cylinder plug (6, 62) and decrease the friction force.

## Description

### BACKGROUND

### 1. Technical Field

The present disclosure relates to a buffer mechanism of an artificial limb, in particular, to a buffer mechanism of an artificial limb adapted to an artificial knee joint, ankle joint, and so on.

### 2. Description of Related Art

If the artificial limb such as an artificial knee joint or an artificial ankle joint is incapable of providing the user with the sufficient buffering capacity when the user is walking, it is possible that the user may kneel down suddenly when the artificial joint produces a certain degree of curvature. In order to prevent the user wearing the artificial limb from kneeling down suddenly as the artificial joint fails to produce the sufficient buffering capacity, the connecting rod mechanism of the artificial joint is usually disposed with a buffer cylinder to provide the artificial joint with the sufficient buffering capacity for avoiding the user kneeling down when the artificial joint produces a certain degree of curvature. In addition, the buffer cylinder also provides the artificial joint with the reverse thrust to enable the artificial joint to return to the initial state which may be the vertical position when the artificial joint has stopped bending.

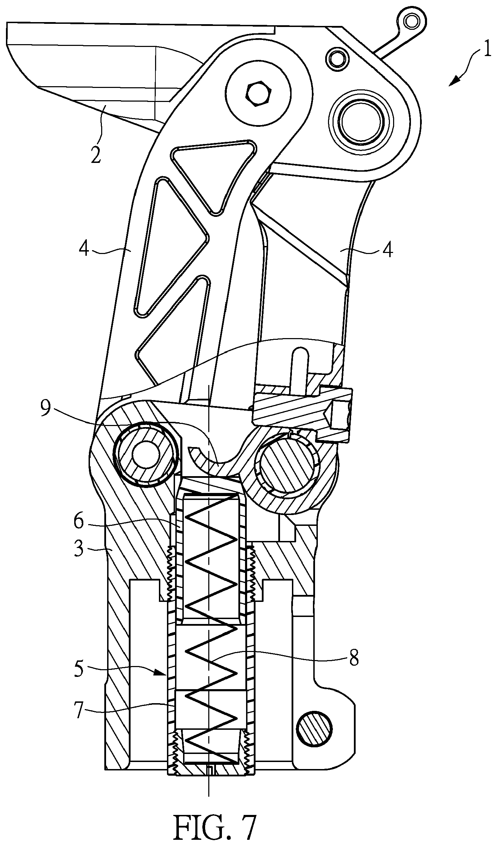

Please refer to FIG. 7 which is a sectional view of the buffer mechanism of the conventional artificial limb. The joint device 1 of the buffer mechanism of the conventional artificial limb includes a joint connecting base 2, an artificial limb connecting base 3, a plurality of connecting rods 4 disposed between the joint connecting base 2 and the artificial limb connecting base 3, and a buffer cylinder 5. The buffer cylinder 5 includes a cylinder plug 6 and a sleeve 7. One end of the cylinder plug 6 is exposed outside the sleeve 7, and a spring 8 is disposed between the cylinder plug 6 and the sleeve 7. The cylinder plug 6 of the buffer cylinder 5 is exposed at one end of the sleeve 7, and an arc-shaped extending arm 9 protrudes and is formed at one side of one of the connecting rods 4 and close to the plunger cylinder 6. The extending arm 9 has an arc-shaped bottom surface, and abuts against a top surface of the cylinder plug 6 by the arc-shaped bottom surface. When the artificial joint produces a degree of curvature, the arc-shaped bottom surface of the extending arm 9 abuts against the top surface of the cylinder plug 6 to press the cylinder plug 6, thereby pressing the spring 8. Thus the elastic force generated by the spring 8 can provide the artificial joint with the buffering capacity to prevent the user from keeling down suddenly when the artificial joint produces a certain degree of curvature. In addition, the reaction force is provided to enable the cylinder plug 6 to abut against the extending arm 9, thereby enabling the extending arm 9 to push the plurality of connecting rods 4 to return to the vertical position when the artificial joint stops curving.

The technical problem of the buffer mechanism of the conventional artificial joint is that there is the friction force between the arc-shaped bottom surface of the extending arm 9 and the top surface of the cylinder plug 6 when they contact to each other. Hence, when the artificial joint moves, the arc-shaped bottom surface of the extending arm 9 rubs against the top surface of the cylinder plug 6 back and forth, causing the top surface of the cylinder plug 6 to suffer damage and the buffer cylinder 5 has to be replaced after long-term usage. In addition, the friction force negatively affects the movement of the artificial joint and causes the artificial joint to not work smoothly.

In view of this, a buffer mechanism of an artificial limb is provided to overcome the shortcomings mentioned above.

### SUMMARY

The primary purpose of the present disclosure provides a buffer mechanism of an artificial limb to reduce the wear rate of the cylinder plug of the buffer cylinder of the conventional artificial joint so as to enable the artificial joint to work more smoothly.

According to one exemplary embodiment of the present disclosure, a buffer mechanism of an artificial limb is provided, including a joint device, and the joint device includes a joint connecting base, an artificial limb connecting base, a first connecting rod and a second connecting rod. Two sides of the joint device are respectively defined as a front side and a rear side. Two ends of the first connecting rod are respectively pivotally connected to a front side of the joint connecting base and a front side of the artificial limb connecting base, and two sides of the second connecting rod are respectively pivotally connected to a rear side of the joint device and a rear side of the artificial limb connecting base. The joint connecting base and the artificial limb connecting base are connected to each other by the first connecting rod and the second connecting rod, and are able to move back and forth from a vertical to an obtuse angle or from an obtuse angle to vertical by the first connecting rod and the second connecting rod. When the joint device moves from the vertical angel to the curved angle, the first connecting rod moves to the rear side of the joint device. A buffer cylinder is disposed on the artificial limb connecting base and between the first connecting rod and the second connecting rod, and has a sleeve, a cylinder plug and a spring. The cylinder plug is detachably disposed in the sleeve, and the spring is disposed between the sleeve and the cylinder plug. When the cylinder plug moves to the sleeve, the spring is pressed. One end face of the cylinder plug faces the first connecting rod and the second connecting rod. A roller arm protrudes and is disposed at a side surface of the first connecting rod facing the second connecting rod, an end of the roller arm extends to an upper end face of the cylinder plug, a roller is pivotally connected at the end of the roller arm, and the roller arm contacts the end face of the cylinder plug by the roller.

To sum up, the first connecting rod and the cylinder plug of the buffer cylinder provided by the present disclosure are to contact with each other by the roller disposed at the end of the roller arm. When the joint device produces a degree of curvature or returns to the vertical position, the roller disposed at the end of the roller arm contacts the end face of the cylinder plug in the form of rolling to avoid the roller arm and the roller rubbing against the end face of the cylinder plug, thereby reducing the wear rate of the end face of the cylinder plug and decreasing the friction force between the roller arm and the cylinder plug so as to enable the joint device to work more smoothly.

In order to further understand the techniques, means and effects of the present disclosure, the following detailed descriptions and appended drawings are hereby referred to, such that, and through which, the purposes, features and aspects of the present disclosure can be thoroughly and concretely appreciated; however, the appended drawings are merely provided for reference and illustration, without any intention to be used for limiting the present disclosure.

### BRIEF DESCRIPTION OF THE DRAWINGS

The accompanying drawings are included to provide a further understanding of the present disclosure, and are incorporated in and constitute a part of this specification. The drawings illustrate exemplary embodiments of the present disclosure and, together with the description, serve to explain the principles of the present disclosure.
FIG. 1 is a three-dimensional diagram of a buffer mechanism of an artificial limb of the present disclosure.
FIG. 2 is an exploded view of a buffer mechanism of an artificial limb of the present disclosure.
FIG. 3 is a three-dimensional diagram of the first connecting rod, roller arm and roller of the present disclosure.
FIG. 4 is an exploded view of the first connecting rod, roller arm and roller of the present disclosure.
FIG. 5 is a sectional view of the buffer mechanism of the artificial limb of the present disclosure in a vertical position.
FIG. 6 is a sectional view of the buffer mechanism of the artificial limb of the present disclosure in an obtuse angle.
FIG. 7 is a sectional view of the buffer mechanism of the conventional artificial limb.

### DESCRIPTION OF THE EXEMPLARY EMBODIMENTS

Reference will now be made in detail to the exemplary embodiments of the present disclosure, examples of which are illustrated in the accompanying drawings. Wherever possible, the same reference numbers are used in the drawings and the description to refer to the same or like parts.

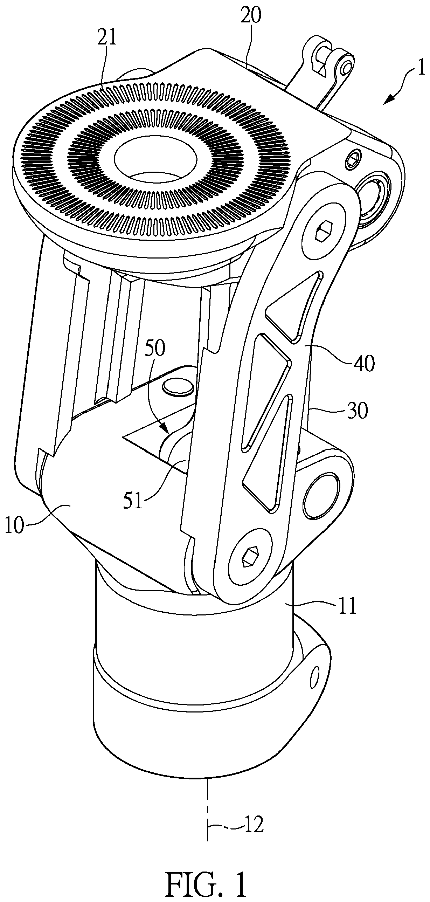

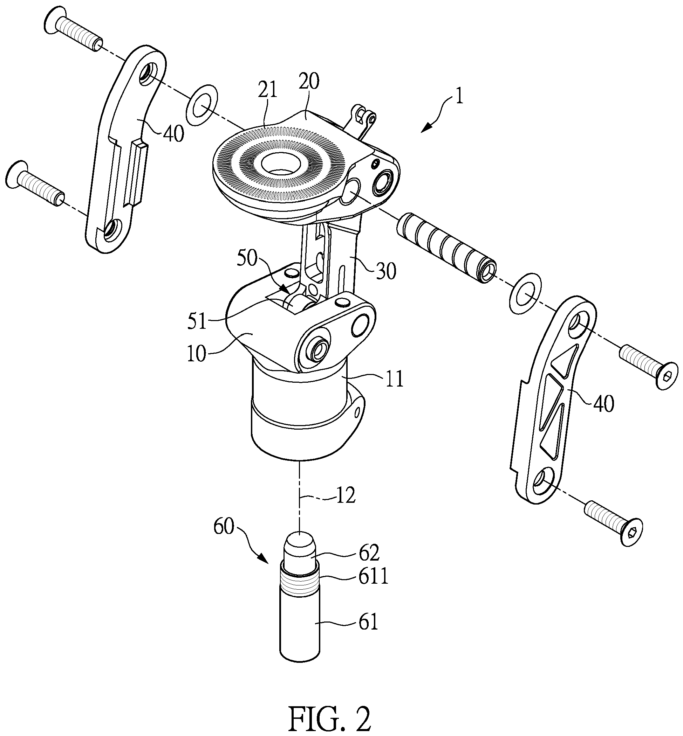

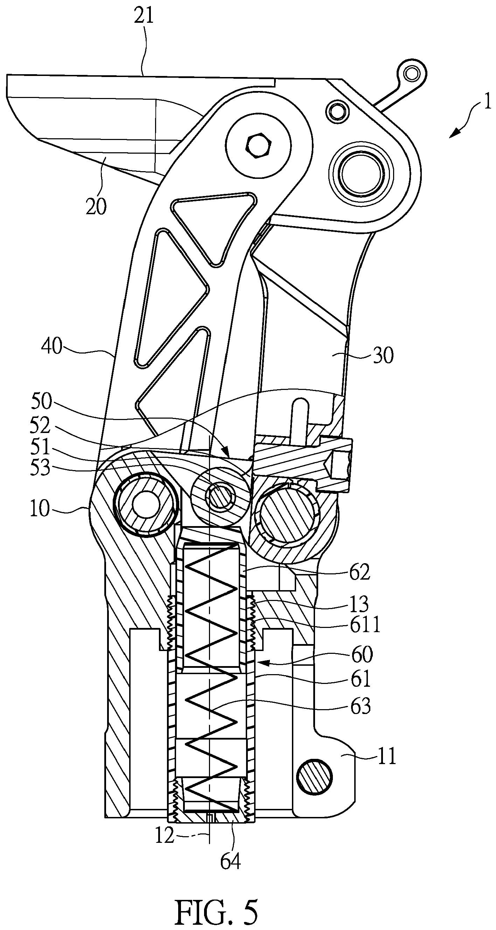

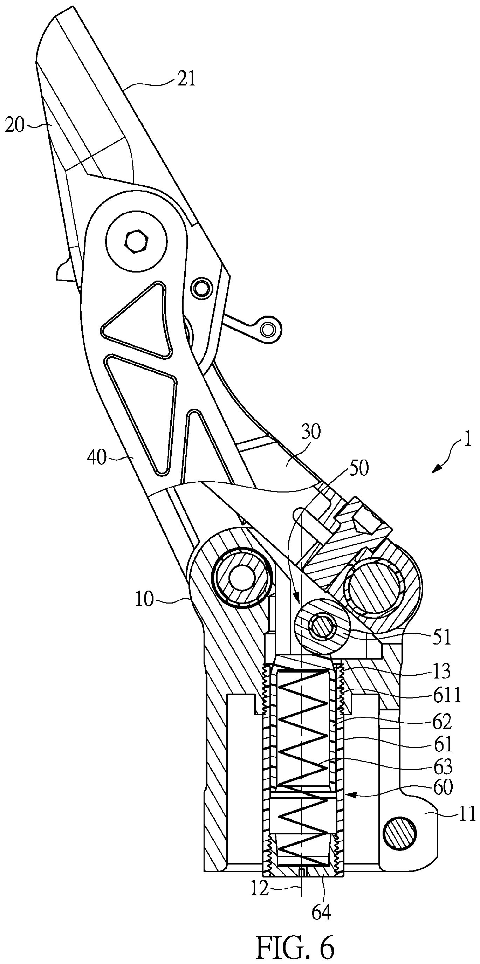

Please refer to FIG. 1 and FIG. 2, which respectively are a three-dimensional diagram of a buffer mechanism of an artificial limb of the present disclosure and an exploded view of a buffer mechanism of an artificial limb of the present disclosure. As shown in the figures, a buffer mechanism of an artificial limb of the present disclosure includes a joint device 1, a buffer cylinder 60 and a roller arm 50, wherein the joint device 1 includes an artificial limb connecting base 10, a joint connecting base 20, a first connecting rod 30 and a second connecting rod 40. Please refer to FIG. 5 and FIG. 6. The right side of the joint device 1 shown in the Figures is defined as a front side, and the left side of the joint device 1 is defined as a rear side. An upper end and a lower end of the first connecting rod 30 are respectively pivotally connected to a front side of the joint connecting base 20 and a front side of the artificial limb connecting base 10. An upper end and a lower end of the second connecting rod 40 are respectively pivotally connected to a rear side of the joint connecting base 20 and a rear side of the artificial limb connecting base 10. Thus the joint connecting base 20 and the artificial limb connecting base 10 are connected with each other by the first connecting rod 30 and the second connecting rod 40, and form a four-bar mechanism.

In the present disclosure, the joint device 1 is an artificial knee joint. In the other embodiments, the joint device 1 may be another artificial joint such as an artificial ankle joint, an artificial wrist joint, and so on. An artificial knee joint is used as an example in the present embodiment, wherein the joint connecting base 20 has a joint bearing surface 21 used to connect with a part of the thigh, and the artificial limb connecting base 10 has a clamping portion 11 used to connect to the calf. In addition, the artificial limb connecting base 10 has a center datum line 12 coinciding with the center of the calf. The joint bearing surface 21 of the joint connecting base 20 is perpendicular to the center datum line 12, and the first connecting rod 30 and the second connecting rod 40 are respectively pivotally connected to the artificial limb connecting base 10 at two sides of the center datum line 12.

Please refer to FIG. 5 and FIG. 6 together. The joint device 1 can produce a degree of curvature. When the joint device 1 produces a degree of curvature, the joint connecting base 20 and the artificial limb connecting base 10 respectively use the first connecting rod 30 and the second connecting rod 40 as an oscillating arm to move relative to each other. As shown in FIG. 5, the joint connecting base 20 and the artificial limb connecting base 10 are at a vertical position before the joint device 1 curves. In addition, the joint connecting base 20 is perpendicular to the center datum line 12 of the artificial limb connecting base 10, the joint bearing surface 21 of the joint connecting base 20 is perpendicular to the center datum line 12, and the first connecting rod 30 and the second connecting rod 40 are parallel to each other. As shown in FIG. 6, the joint connecting base 20 and the artificial limb connecting base 10 move to an obtuse angle when the joint device 1 produces a degree of curvature. When the joint device 1 moves to a degree of curvature, the joint bearing surface 21 and the center datum line 12 are in an inclined state, thereby enabling the thigh connected to the joint connecting base 20 and the calf connected to the artificial limb connecting base 10 to be in an inclined state.

As shown in FIG. 5 and FIG. 6, when the user is walking using the artificial limb, the joint device 1 produces a degree of curvature which is similar to the obtuse angle of a human knee, and the joint connecting base 20 and the artificial limb connecting base 10 are respectively driven to move back and forth from the vertical position to the obtuse angle or from the obtuse angle to the vertical position by the thigh connected to the joint connecting base 20 and the calf connected to the artificial limb connecting base 10. In addition, when the joint device 1 moves from the vertical position to the obtuse angle, the top of the first connecting rod 30 moves to the rear side of the joint device 1.

The buffer cylinder 60 is disposed on the artificial limb connecting base 10. On the one hand, when the joint device 1 produces a degree of curvature, the buffer cylinder 60 provides the joint device 1 with a buffering capacity to prevent the user from kneeling down suddenly while walking. On the other hand, the buffer cylinder 60 provides the joint device 1 with a push force to push the joint device 1 to return to the vertical position from the obtuse angle when the joint device 1 has stopped bending. After that, the joint connecting base 20 and the artificial limb connecting base 10 are in the vertical position as shown in FIG. 5.

Please refer to FIG. 2, FIG. 5 and FIG. 6 together. The buffer cylinder 60 is disposed on the artificial limb connecting base 10 and between the first connecting rod 30 and the second connecting rod 40. The buffer cylinder 60 includes a sleeve 61, a cylinder plug 62 and a spring 63. The sleeve 61 is a cylindrical body, one end of the sleeve 61 has an opening, the cylinder plug 62 is detachably disposed in the sleeve 61, and the other end of the sleeve 61 opposite to the cylinder plug 62 is disposed with a bottom plug 64. The bottom plug 64 is fixed at a bottom of the sleeve 61 by means of screwing, and the spring 63 is disposed between the cylinder plug 62 and the bottom plug 64.

As shown in FIG. 5 and FIG. 6, a top end of the cylinder plug 62 protrudes beyond a top end of the sleeve 61, and the protrusion of the cylinder plug 62 contacts a roller 51 disposed at an end of the roller arm 50 and is pressed by the roller arm 50 and the roller 51, such that the cylinder plug 62 is pressed to be close to the sleeve 61 so as to press the spring 63 to produce a compression action.

Please refer to FIG. 5 and FIG. 6 again. The buffer cylinder 60 has an adjustable spring force, and the adjustable spring force is described as follows. Two ends of the spring 63 of the buffer cylinder 60 respectively abut against the cylinder plug 62 and the bottom plug 64. Here, the bottom plug 64 is fixed at the bottom of the sleeve 61 by means of screwing. When the user rotates the bottom plug 64, the bottom plug 64 moves along the central axial line of the sleeve 61 to change the distance between the bottom plug 64 and the cylinder plug 62, thereby making the spring 63 have different degrees of compression to change the elastic coefficient so as to adjust the elastic force of the spring 63 accordingly.

An external side of the opening of the sleeve 61 of the buffer cylinder 60 in which the cylinder plug 62 is inserted is disposed with an external thread portion 611, an internal thread hole 13 is disposed on the artificial limb connecting base 10, and the center of the internal thread hole 13 is parallel to the center datum line 12. A diameter of the internal thread hole 13 is the same as a diameter of the external thread portion 611 of the sleeve 61, so that the sleeve 61 can be fixed in the internal thread hole 13 by the external thread portion 611, thereby enabling the buffer cylinder 60 to be disposed on the artificial limb connecting base 10.

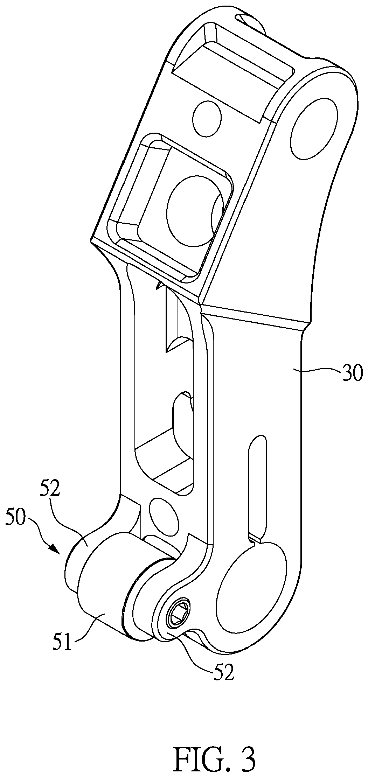

As shown in FIG. 3 to FIG. 6, the roller arm 50 protrudes and is disposed at a side surface of the first connecting rod 30 facing the second connecting rod 40, an end of the roller arm 50 extends to an upper end face of the cylinder plug 62, and the roller 51 is pivotally connected at the end of the roller arm 50, and the roller arm 50 contacts the end face of the cylinder plug 62 by means of the roller 51.

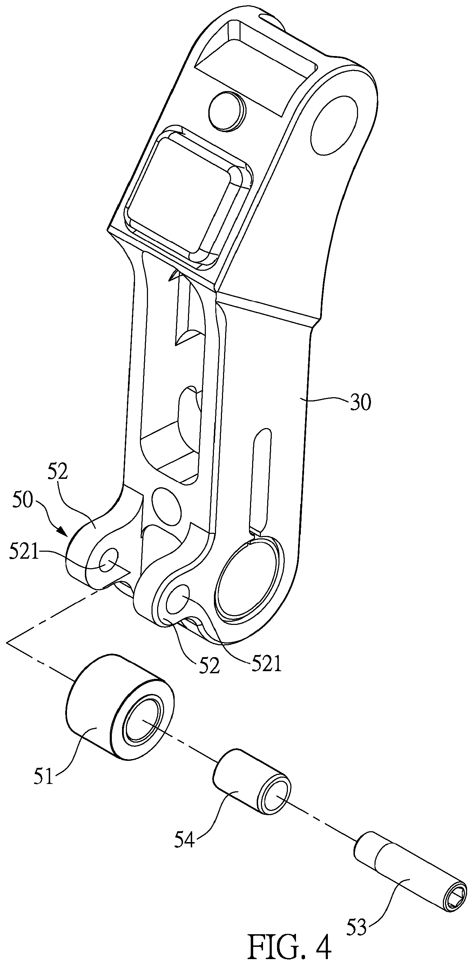

As shown in FIG. 4, in the present embodiment, the roller arm 50 includes a pair of roller supports 52 respectively disposed at two side surfaces of the first connecting rod 30. The pair of roller supports 52 is parallel to each other and has a gap therebetween, and is perpendicular to the side surface of the first connecting rod 30. The roller 51 is disposed between the pair of roller supports 52. A pair of shaft holes 521 are respectively disposed in the center of the pair of roller supports 52 corresponding to the roller 51 and configured to enable a roll axis 53 to penetrate the pair of shaft holes 521 and the center of the roller 52, thereby pivotally connecting the roller 51 between the ends of the pair of roller supports 52. In addition, a bushing 54 is disposed between an external side of the roll axis 53 and the roller 51. The bushing 54 is made of a high-molecular weight material characterized by a low friction coefficient and abrasion resistance, thereby reducing the frictional resistance when the roller 51 is rotating with respect to the roll axis 53.

As shown in FIG. 5 and FIG. 6, the roller arm 50 is substantially perpendicular to the first connecting rod 30 and the center of the roller 51 deviates from a side surface of the first connecting rod 30 to the rear side of the joint device 1. When the first connecting rod 30 moves to the rear side of the joint device 1, the roller arm 50 and the roller 51 move together with the first connecting rod 30 to move counterclockwise based on a rotation axis where the bottom of the first connecting rod 30 is pivotally connected to the artificial limb connecting base 10.

When the joint device 1 of the present disclosure produces a degree of curvature and the joint connecting base 20 and the artificial limb connecting base 10 move from the vertical position to the obtuse angle, the roller arm 50 and the roller 51 are driven to move by the first connecting rod 30 based on a rotation axis where the bottom of the first connecting rod 30 is pivotally connected to the artificial limb connecting base 10, and the roller 51 is pressed downwardly to abut against the top surface of the cylinder plug 62 to enable the cylinder plug 62 to press the spring 62. Thus the spring 63 provides the roller 51 and the roller arm 50 with the buffering capacity in the process of the joint connecting base 20 and the artificial limb connecting base 10 moving from the vertical angle to the curved angle, thereby preventing the user from keeling down suddenly when walking.

In addition, when the joint device 1 stops curving, the cylinder plug 62 is pushed by the elastic force generated by the spring 63 to move upwardly to push the roller 51 and the roller arm 50 to move clockwise so as to drive the first connecting rod 30 to return to the vertical position.

In summary, the first connecting rod 30 and the cylinder plug 62 of the buffer cylinder 60 provided by the present disclosure contact with each other by the roller 51 disposed at the end of the roller arm 50. When the joint device 1 produces a degree of curvature or returns to the vertical position, the roller 51 disposed at the end of the roller arm 50 contacts the end face of the cylinder plug 62 in the form of rolling to avoid the roller arm 50 and the roller 51 rubbing against the end face of the cylinder plug 62, thereby reducing the wear rate of the end face of the cylinder plug 62 and decreasing the friction force between the roller arm 50 and the cylinder plug 62 so as to enable the joint device 1 to work more smoothly.

The above-mentioned descriptions represent merely the exemplary embodiment of the present disclosure, without any intention to limit the scope of the present disclosure thereto. Various equivalent changes, alterations or modifications based on the claims of present disclosure are all consequently viewed as being embraced by the scope of the present disclosure.

## Claims

1. A buffer mechanism of an artificial limb, comprising:
a joint device (1) comprising a joint connecting base (20), an artificial limb connecting base (10), a first connecting rod (30) and a second connecting rod (40); two sides of the joint device (1) respectively defined as a front side and a rear side; two ends of the first connecting rod (30) respectively pivotally connected to a front side of the joint connecting base (20) and a front side of the artificial limb connecting base (10) and two ends of the second connecting rod (40) respectively pivotally connected to a rear side of the joint device (1) and a rear side of the artificial limb connecting base (10); the joint connecting base (20) and the artificial limb connecting base (10) connected to each other by the first connecting rod (30) and the second connecting rod (40) and moving relative to each other from a vertical position to an obtuse angle or from an obtuse angle to a vertical position, and when the joint device (1) moves from the vertical position to the obtuse angle, the first connecting rod (30) moving to the rear side of the joint device (1);
a buffer cylinder (5, 60) disposed on the artificial limb connecting base (10) and between the first connecting rod (30) and the second connecting rod (40); the buffer cylinder (5, 60) comprising a sleeve (7, 61), a cylinder plug (6, 62) and a spring (8, 63); the cylinder plug (6, 62) detachably disposed in the sleeve (7, 61), the spring (8, 63) disposed in the sleeve (7, 61) and the cylinder plug (6, 62); when the cylinder plug (6, 62) moves into the sleeve (7, 61), the spring (8, 63) being pressed, and an end face of the cylinder plug (6, 62) facing the first connecting rod (30) and the second connecting rod (40); and
a roller arm (50) protruding and disposed at a side surface of the first connecting rod (30) facing the second connecting rod (40), an end of the roller arm (50) extending to an upper end face of the cylinder plug (6, 62), and an end of the roller arm (50) pivotally connected to a roller (51), and the roller arm (50) contacting the end face of the cylinder plug (6, 62) by the roller (51).

2. The buffer mechanism according to claim 1, wherein the roller arm (50) comprises a pair of roller supports (52) respectively disposed at two sides of the first connecting rod (30), the pair of roller supports (52) are parallel to each other and have a gap therebetween, and perpendicular to a side surface of the first connecting rod (30), the roller is disposed between the pair of roller supports (52), a pair of shaft holes (521) is respectively disposed in a center of the pair of roller supports (52) corresponding to the roller (51) and configured to enable a roll axis (53) to penetrate the pair of shaft holes (521) and the center of the roller (51), thereby pivotally connecting the roller (51) between the ends of the pair of roller supports (52).

3. The buffer mechanism according to claim 2, wherein a bushing (54) is disposed between an external side of the roll axis (53) and the roller (51).

4. The buffer mechanism according to claim 3, wherein the artificial limb connecting base (10) has a center datum line (12), a central axial line of the sleeve (7, 61) and a central axial line of the cylinder plug (6, 62) are parallel to the center datum line (12); the first connecting rod (30) and the second connecting rod (40) are respectively pivotally connected to the artificial limb connecting base (10) at two sides of the center datum line (12); the joint connecting base (20) has a joint bearing surface (21); when the joint connecting base (20) and the artificial limb connecting base (10) are in a vertical position, the joint bearing surface (21) is perpendicular to the center datum line (12), and when the joint connecting base (20) and the artificial limb connecting base (10) are in an obtuse angle, the joint bearing surface (21) and the center datum line (12) are in an inclined state.

5. The buffer mechanism according to claim 4, wherein an internal thread hole (13) is disposed between the first connecting rod (30) and the second connecting rod (40), a center of the internal thread hole (13) is parallel to the center datum line (12); one end of the sleeve (7, 61) in which the cylinder plug (6, 62) is inserted is disposed with an external thread portion (611), and the internal thread hole (13) and the external thread portion (611) are fixed to each other to enable the sleeve (7, 61) to be fixed in the internal thread hole (13) by the external thread portion (611).

6. The buffer mechanism according to claim 5, wherein an end of the sleeve (7, 61) opposite to the cylinder plug (6, 62) is disposed with a bottom plug (64) and the spring (8, 63) is disposed in the cylinder plug (6, 62) and the bottom plug (64).

7. The buffer mechanism according to claim 6, wherein the bottom plug (64) is fixed at a bottom of the sleeve (7, 61) by means of screwing, and when rotating the bottom plug (64), the bottom plug (64) moves along the central axial line of the sleeve (7, 61) to adjust a position thereof, thereby adjusting the elastic force generated by the spring (8, 63).
